# EUROPEAN PATENT APPLICATION

(11) **EP 0 571 116 A1**
(43) Date of publication of application: **24.11.1993**
(21) Application number: 93303591.7
(22) Date of filing: 10.05.1993
(51) Int. Cl.: B01L 3/14

(54) **Blood collection tube assembly**

(30) Priority: 13.05.1992 US 882194
(71) Applicant: Becton Dickinson and Company, Franklin Lakes, New Jersey 07417-1880 (US)
(72) Inventor: Grippi, Nicholas A., Ramsey, New Jersey 07446 (US); Swaim, David, Leonia, New Jersey 07605 (US)
(74) Representative: Ruffles, Graham Keith

(57) **Abstract**

The present invention is a plastic container (42) with a barrier label (45). In use, the barrier label (45) is wrapped around and adhered to a container (42). The barrier label (45) preferably is transparent so that the contents of the container (42) are visible to the observer at the same time identifying information may be displayed on the barrier label (45). The barrier label is useful for providing an effective barrier against gas permeability in containers and for extending shelf-life of containers, especially plastic evacuated blood collection devices.

## Description

### BACKGROUND OF THE INVENTION

### 1. Field of the Invention

This invention relates to a barrier label for providing an effective barrier against gas and water vapor permeability of containers and for extending shelf-life of containers, especially plastic evacuated blood collection tubes.

### 2. Description of the Related Art

With the increased emphasis on the use of plastic medical products, a special need exists for improving the barrier properties of articles made of polymers.

Such medical products that would derive a considerable benefit from improving their barrier properties include, but are not limited to, collection tubes and particularly those used for blood collection.

Blood collection tubes require certain performance standards to be acceptable for use in medical applications. Such performance standards include the ability to maintain greater than about 90% original draw volume over a one year period, to be radiation sterilizable and to be noninterfering in tests and analysis.

A critical performance standard of the blood collection tube is the draw volume retention. Draw volume retention depends on the existence of a partial vacuum, or reduced pressure, inside the tube. The draw volume changes in direct proportion to the change in vacuum (reduced pressure). Therefore, draw volume retention is dependent on good vacuum retention.

For example, it is important to maintain the vacuum over a period of time in order to provide appropriate storage life for those tubes prior to their being used. That is, it is important for the vacuum level to be maintained for a period of time prior to the time when a technician or a nurse uses the tube for collecting a blood sample.

Radiation sterilizable means that after the tube is exposed to certain levels of radiation in the sterilization process, there is substantially no change in optical or mechanical and functional properties.

Noninterference in performance of a tube's specifications means that the materials of the tube, such as glass or plastic materials do not interfere with testing and analysis that is typically performed on blood in the tube. Such tests include but are not limited to hematology, blood chemistry, blood typing, toxicology analysis and therapeutic drug monitoring. Furthermore, the tube must be capable of being subjected to automated machinery such as centrifuges.

Typically, plastic evacuated blood collection tubes have small paper labels applied over the least amount of outer surface area of the tube. However, these small paper labels do not contribute to improving the barrier properties of the plastic tube. Their intended purpose is for identification of the tube.

Despite the variety of known labels there are apparently no labels specifically made or used as barrier labels which can be attached conveniently and securely to the outer surface of a tube, such as a plastic evacuated blood collection tube, while not obscuring its contents so that the tube with the barrier label would be able to meet certain performance standards and be effective and usable in medical applications.

### SUMMARY OF THE INVENTION

The present invention is a plastic composite container with a barrier label disposed over the outer surface of the composite container. That is, a barrier label is secured over the previously formed plastic container. Such a barrier label improves the barrier properties of plastic containers and extends the shelf-life of plastic containers, especially plastic evacuated blood collection devices.

The barrier label may be placed or formed around the container and then adhered to the container by methods such as, but not limited to, an adhesive layer, heating the barrier label and container to a temperature sufficient to cause the barrier label to heat-shrink onto the container or by expanding the container into the barrier label by blow molding.

Desirably the barrier label of the present invention is a polymer film with a chemical compound coating which may be deposited by a non-reactive ion plating, plasma enhanced chemical vapor deposition, or magnetron sputtering method. The chemical coating may be an oxide, a metal oxide or a rare earth element. Most preferably, the chemical compound coating is silicon oxide based.

Suitable films to be coated for use as barrier labels according to the present invention include, but are not limited to polypropylene films, low and high density polythene films and polyvinylchloride films.

Preferably, the barrier label coated with a silicon oxide based - compound provides a transparent, translucent or colorless appearance and may have printed matter applied thereon.

Barrier labels coated with the silicon oxide-based coating are able to maintain far better vacuum retention and draw volume retention than previous barrier labels comprised of polymer compositions and blends thereof. Most notably is the clarity of the coated film and its durability to substantially withstand resistance to impact.

Printing may be placed on the barrier label which is to be wrapped around the container of interest. For example, a product identification, bar code, brand name, company logo, lot number, expiration date and other data and information may all be included on the label surface. Moreover, a matte finish or a corona discharged surface may be developed on the outer surface of the barrier label so as to make the surface appropriate for writing additional information on the label. Furthermore, another pressure sensitive adhesive label may be placed over the outer surface of the barrier label so as to accommodate various hospital over-labels, for example.

It may be appropriate to apply a heat-activated or pressure sensitive adhesive to the inner surface of the barrier label. The adhesive may be printed or applied to the inner surface of the barrier label. A heat-activated adhesive may be applied when the barrier label is heated for application onto the container. In this way the adhesive becomes activated and helps adhere the barrier label to the container being wrapped. Any conventional heat-activated or pressure sensitive adhesive may be used for application onto the container and preferably does not interfere with transparency of the barrier label.

A unique feature of the invention is that a plastic container with the barrier label has improved barrier properties including, but not limited to, the reduction of permeating gas molecules and water vapor into and out of the container.

### DESCRIPTION OF THE DRAWINGS

FIG. 1 is a perspective view of a typical blood collection tube with a stopper.

FIG. 2 is a longitudinal sectional view of the tube of FIG. 1 taken along line 2-2 thereof.

FIG. 3 is a longitudinal sectional view of a tube-shaped container without a stopper similar to the tube of FIG. 1 with the barrier label thereover it, and illustrating one form of the invention.

FIG. 4 is a longitudinal sectional view of a tube-shaped container without a stopper similar to the tube of FIG. 1 with a barrier label thereover illustrating an additional embodiment of the invention.

### DETAILED DESCRIPTION

The present invention may be embodied in other specific forms and is not limited to any specific embodiment described in detail which is merely exemplary. Various other modifications will be apparent to and readily made by those skilled in the art without departing from the scope and spirit of the invention. The scope of the invention will be measured by the appended claims and their equivalents.

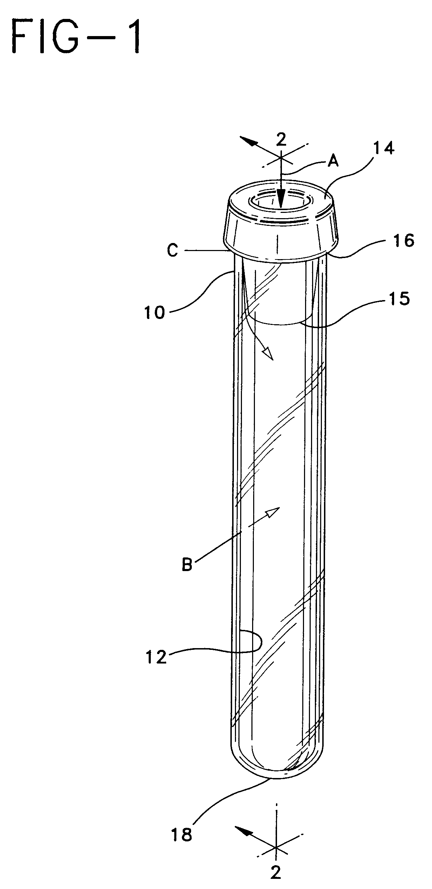

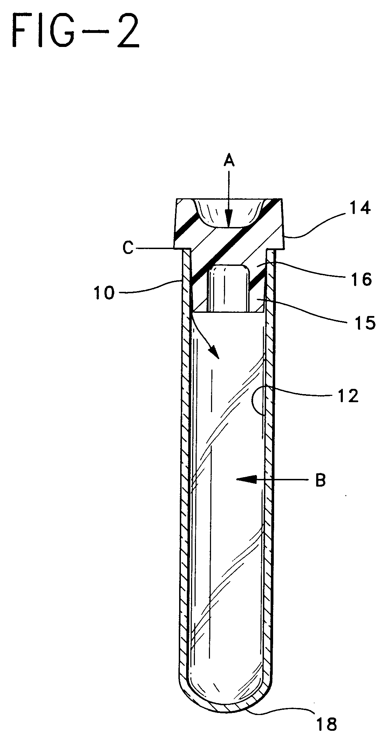

Referring to the drawings in which like reference characters refer to like parts throughout the several views thereof, FIGS. 1 and 2 show a typical blood collection tube **10,** having anopen end **16**, a closed end **18** and stopper **14** which includes a lower annular portion or skirt **15** which extends into and presses against the inside walls **12** of the tube for maintaining stopper **14** in place.

FIG. 2 schematically illustrates that there are three mechanisms for a change in vacuum in a blood collection tube: (A) gas permeation through the stopper material; (B) gas permeation through the tube material and (C) leak at the closure-tube interface. Therefore, when there is substantially no gas permeation and no leak, there is good vacuum retention and good draw volume retention.

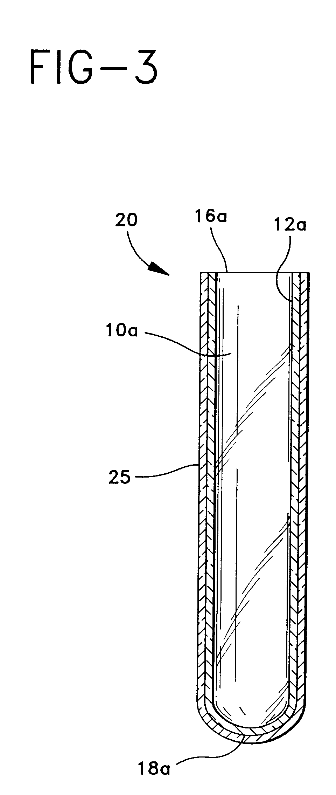

FIG. 3 shows the preferred embodiment of the invention, a plastic tube with a barrier label comprising a film coated with a silicon oxide based composition. The preferred embodiment includes many components which are substantially identical to the components of FIGS. 1 and 2. Accordingly, similar components performing similar functions will be numbered identically to those components of FIGS. 1 and 2, except that a suffix "a" will be used to identify those components in FIG. 3.

Referring now to FIG. 3, the preferred embodiment of the invention, collection tube assembly **20** comprises a plastic tube **10a**, having an open end **16a** and a closed end **18a**. This embodiment shows a barrier label **25** extending over a substantial portion of the length of the tube. As shown in FIG. 3, barrier label **25** ends at edge **26**. Alternatively, the barrier label can cover all of the tube with the exception of open end **16a** thereof.

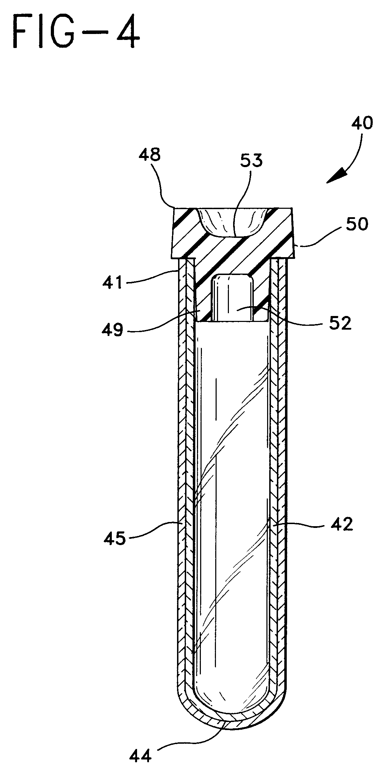

FIG. 4 illustrates an alternate embodiment of the invention, wherein collection tube assembly **40** comprises stopper **48** in place for closing open end **41** of tube **42**. As can be seen, stopper **48** includes an annular upper portion **50** which extends over the top edge of tube **42** as well as the top edge of film **45**. Stopper **48** includes a lower annular portion or skirt **49** which extends into and- presses against the inside walls of tube **42** for maintaining stopper **48** in place. Also, the annular lower skirt **49** of stopper **48** defines a well **52** which, in turn, defines a septum portion **53** of stopper **48** for receiving a cannula therethrough. Thus, the user, once receiving a container such as that shown in FIG. 4 with a sample contained therein, may insert a cannula through septum **53** in known fashion, for receiving part or all of the contents in tube **42** to perform various tests on a sample. Covering a substantial portion of the length of the tube is film **45**. Film **45** covers substantially all of the tube with the exception of open end **41** thereof.

It will be understood by practitioners-in-the-art, that such tubes may contain reagents in the form of additives or coatings on the inner wall of the tube or in a solution near or at the inside closed end of the tube. FIG. 4 differs from the embodiment in FIG. 3 in that the tube is first evacuated with the simultaneous placement of stopper **48** therein followed by the application of film **45** thereover the tube. Alternatively, the barrier label can cover all of the tube with the exception of the stopper.

An alternate embodiment of the invention also includes a barrier label incorporating both the upper portion of the stopper, as well as the entire container tube. Such an embodiment may be utilized, for example, for sealing the container with the stopper in place. Once a sample has been placed in the tube, the sample cannot be tampered with by removal of the stopper. Additionally, serrations could be included at the tube, stopper interface. The serrations may be registered so that it can be determined if the sealed container has been tampered with.

The barrier label is desirably a film coated with a high barrier performance material such as silicon oxide. Preferred film materials include, but are not limited to, polymeric substrate resins. Polymeric substrate resins include, but are not limited to polyamide, polyolefin and polyester. Polyamide includes but is not limited to, biaxial oriented nylon, aromatic amorphous polyamide, polyvinyl chloride and mixtures thereof. Polyolefin includes, but is not limited to biaxial oriented polypropylene, low density polyethylene, polychlorotrifluoroethylene and mixtures thereof. Polyester includes, but is not limited to, polyethylene terephthalate, polyethylene naphthalate, polyethylene isophthalate and mixtures thereof.

Methods for depositing an oxide based coating on a film include ion plating, plasma enhanced vapor deposition, plasma polymerization, and sputtering as described in European Publication Numbers 0 113 555, 0 469 926, 0 299 754, and U.S. Patent Nos. 5,047,131, 4,888,199, 4,847,469, the disclosures of which are herein all incorporated by reference.

The deposition of an oxide based coating by an ion plating or magnetron sputtering method enables relatively large crystals to be formed in the coating, such crystals provide a barrier to the transmission of water vapor, oxygen or other gases. The film coating may include metal atoms having a relatively high atomic number such as lead, silica, indium and tin or aluminum.

A plastic blood collection tube with a barrier label preferably coated with a silicon oxide based coating may effectively be used in such applications as routine chemical analysis, biological inertness, hematology, blood chemistry, blood typing, toxicology analysis or therapeutic drug monitoring and other clinical tests involving body fluids.

The silicon oxide based film or blends thereof used in accordance with this disclosure, may contain conventional additives and ingredients which do not adversely affect the properties of articles made therefrom.

It will be understood that the barrier label of the invention herein may arranged to conform to any configuration of container and the stopper or cap therefor. Thus, any form of a container wherein plastic is the preferred material may be used. The barrier label is particularly directed to increasing the integrity of a plastic container and for increasing the integrity of other containers made of glass and paper as well.

## Claims

1. A sample container assembly comprising:
a plastic container having an open end, a closed end, an inner surface and an outer surface; and
a barrier label comprising a polymer film coated with an oxide-based composition, associated over the outer surface of said container and extending over a major portion of said outer surface of said outer surface of said container.

2. The assembly of Claim 1 further comprising a closure in said open end of said container whereby a container and closure interface is formed.

3. The assembly of Claim 2 wherein said oxide based composition is selected from the group consisting of aluminum, silica, tin and indium.

4. The assembly of Claim 3 wherein said plastic container is a tube and said closure is an elastomeric stopper.

5. The assembly of Claim 4 wherein said tube is evacuated.

6. The assembly of Claim 5 wherein said barrier label comprises a polymer film material selected from the group consisting of polyamide, polyolefin and polyester.

7. The assembly of Claim 6 wherein said oxide-based composition of said barrier label is silicon oxide.

8. The assembly of Claim 6 wherein said polymer film is a polyamide selected from the group consisting of biaxial oriented nylon, aromatic amorphous polyamide and polyvinyl chloride.

9. The assembly of Claim 6 wherein said polymer film is a polyolefin selected from the group consisting of biaxial oriented polypropylene, low density polyethylene and polychlorotrifluoroethylene.

10. The assembly of Claim 6 wherein said polymer film is a polyester selected from the group consisting of polyethylene terephthalate, polyethylene naphthalate and polyethylene isophthalate.

11. The assembly of Claim 10 wherein said oxide based composition of said barrier label has been deposited by an ion plating, plasma enhanced vapor deposition, plasma polymerization or sputtering.
